Europäisches Patentamt

European Patent Office

Office européen des brevets

(11) Publication number: **0 265 672 B1**

# EUROPEAN PATENT SPECIFICATION

(45) Date of publication of patent specification: **10.03.93**

(51) Int. Cl.⁵: **G01N 33/569**, A61K 37/20, A61K 39/104, //C12N1/20, G01N33/531

(21) Application number: **87113996.0**

(22) Date of filing: **24.09.87**

(54) Method for detection of gram-negative bacteria.

(30) Priority: **26.09.86 US 912439**

(43) Date of publication of application:
**04.05.88 Bulletin 88/18**

(45) Publication of the grant of the patent:
**10.03.93 Bulletin 93/10**

(84) Designated Contracting States:
**AT BE CH DE ES FR GB GR IT LI LU NL SE**

(56) References cited:
**EP-A- 0 050 424**
**EP-A- 0 136 947**
**US-A- 4 587 121**

**EUROPEAN JOURNAL OF BIOCHEMISTRY, Vol. 132, 1983, pages 329-337; P.S.N. ROWE et al.: "Structure of the Core Oligosaccharide from the Lipopolysaccharide of Pseudomonas aeruginosa PACIR and Its Defective Mutants"**

(73) Proprietor: **THE REGENTS OF THE UNIVERSITY OF CALIFORNIA**
**2199 Addison Street**
**Berkeley, California 94720(US)**

(72) Inventor: **Young, Lowell S.**
**2025 Jackson Street**
**San Francisco, CA 94109(US)**
Inventor: **Jackobson, Mark A.**
**1122 El Centro**
**Oakland, CA 94602(US)**

(74) Representative: **Glawe, Delfs, Moll & Partner Patentanwälte**
**Postfach 26 01 62 Liebherrstrasse 20**
**W-8000 München 26 (DE)**

JOURNAL OF IMMUNOLOGICAL METHODS, Vol. 82, 1985, pages 199-207; B.J. APPEL-MELK et al.: "An Enzym-Linked Immunosorbent Assay (ELISA) for the Measurement of Antibodies to Different Parts of the Gram-Negative Lipopolysaccharide Core Region"

CHEMICAL ABSTRACTS, Vol. 87, no. 15, 10th October 1977, abstract no. 116206x, Columbus, Ohio (US); R. KOHLER et al.: "Detection of IgG antibodies to type-specific Pseudomonas aeruginosa lipopolysaccharides by solid-phase radioimmunoassay"

**Description**

BACKGROUND OF THE INVENTION

Field of the Invention

The present invention relates generally to infectious diseases and, more particularly, to the diagnosis of chronic Pseudomonas aeruginosa infection especially as associated with cystic fibrosis.

Cystic fibrosis (CF) is the most common lethal genetic syndrome among Caucasians. In the United States, the incidence is 1 in 2,000 live births. CF is a generalized metabolic disorder involving change in the serous and mucous secretions resulting in, among other symptoms, progressive obstructive pulmonary disease and malabsorption due to pancreatic enzyme insufficiency. As late as the 1950's, life expectancy for cystic fibrosis patients was extremely short. Even today, only half of CF patients reach their 20th birthday.

Pulmonary complications associated with progressive obstructive pulmonary disease are responsible for the majority of morbidity and virtually all mortality in afflicted patients. Pulmonary infections are generally a fact of life for the CF patient. The majority of these infections are caused by the gram-negative bacterium Pseudomonas aeruginosa (PA). Once established in CF patients, PA is an extremely difficult organism to eradicate. The majority of CF patients are chronically infected with PA.

Treatment of chronic PA infection with antibiotics, including the aminoglycosides, generally fails to eradicate PA. Indeed, some researchers believe that the continuous use of antibiotics actually contributes to the bacteria's persistence. It is not uncommon to find patients infected with PA strains resistant to all known antibiotics. These patients often have an unrelenting deterioration of pulmonary function ultimately leading to death. A recent study reported survival rates to age 16 years in CF afflicted children with and without chronic PA infection. The researchers reported a 53% survival rate in infected children versus an 84% survival rate in non-infected children.

Recent advances in the treatment of gram-negative bacterial infections, particularly PA infections, provide hope for CF patients with chronic PA infections. Methods employing monoclonal antibody composition, such as those disclosed in U.S. Patent Application Serial No. 855,878, appear particularly promising. Before any therapy can be effective, however, it is important to accurately diagnose the nature of the infection. Once diagnosed, it is extremely beneficial to follow the effectiveness of therapy throughout its course. There is therefore a need for compositions and methods useful in the diagnosis of chronic PA infection and in following the course of such infection.

Discussion Of The Relevant Literature

U.S. Patent No. 4,587,121 discloses the use of hyperimmune serum globulin having high titers of antibody against all seven Fisher Immunotypes of Pseudomonas aeruginosa for use in treating patients with Pseudomonas infection.

Rowe and Meadow, Eur. J. Biochem. (1983) 132:329-37, report the isolation of lipopolysaccharides from Pseudomonas aeruginosa strain PAC1R and its lipopolysaccharide-defective mutants, including PAC 605.

Appelmelk et al., J. Immuno. Meth. (1985) 82:199-207, describe an enzyme linked immunosorbant assay (ELISA) for the measurement of antibodies to different parts of gram-negative lipopolysaccharide core region employing core antigens of E. coli J5 LPS, Salmonella minnesota R595 LPS or E. coli lipid A.

Rowe and Meadow, supra, as well as Appelmelk et al., supra, do not disclose or suggest a method for detecting chronic P. aeruginosa infections in patients using LPS of the PAC 605 strain as an antigen in an immunoassay in order to determine the anti-PAC 605 LPS antibody titer using donor sera of non-infected or convalescent patients as controls.

Meadow et al., J. Gen. Microbio. (1984) 130:631-44, describe the characterization of surface antigens in Pseudomonas aeruginosa.

Gascon et al., 25th ICAAC, Minneapolis, Minn., (1985) Abstract No. 77, describe the functional activity of monoclonal antibodies against common core lipopolysaccharide antigen of P. aeruginosa.

Poxton et al., FES Microbiology Letter (1985) 27:247-51, describe the association on SDS-polyacrylamide gels of lipopolysaccharide and outer membrane proteins of P. aeruginosa as revealed by monoclonal antibodies and Western blotting employing P. aeruginosa Habs serotypes 1 and 4.

Wilmott et al., AJDC (1985) 139:669-71 report cystic fibrosis survical rates and the influences of allergy and Pseudomonas aeruginosa.

Summary of the Invention

The present invention provides a method useful in the diagnosis and monitoring of Gram-negative infection, especially chronic Pseudomonas aeruginosa infection associated with cystic fibrosis. Pseudomonas aeruginosa strain PAC 605 core lipopolysaccharide determinants are employed as a solid phase antigen in enzyme linked immunosorbant assays and Western blot immunoassay to measure human IgG response to P. aeruginosa infection.

BRIEF DESCRIPTION OF THE FIGURES

Fig. 1 is an immunoblot analysis of patient sera;
Fig. 2 is a silver stain of SDS-PAGE;
Fig. 3 is an immunoblot analysis of patient sera vs. proteinase K digestion and periodate oxidation of PAC 605 LPS; and

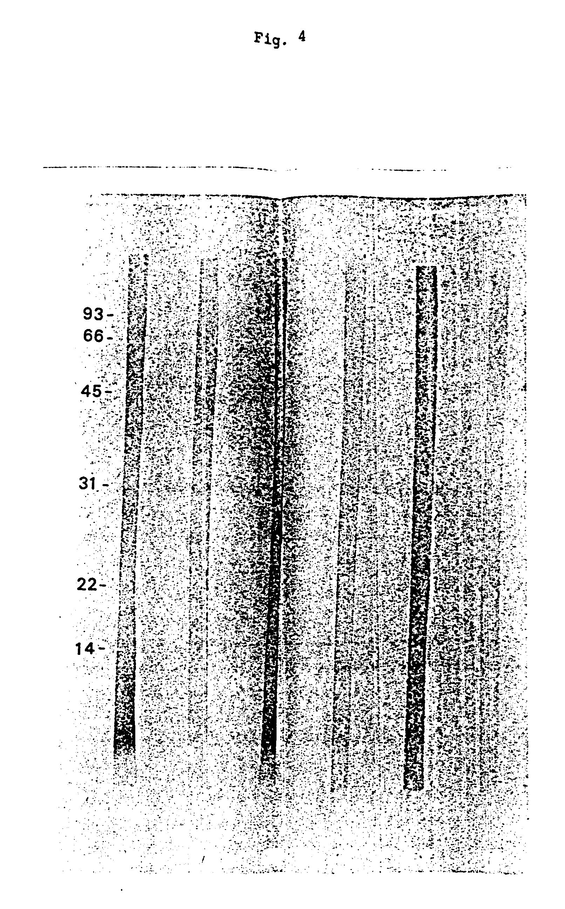

Fig. 4 is an immunoblot analysis of patient sera vs. J5 E. coli LPS.

DETAILED DESCRIPTION OF THE PREFERRED EMBODIMENTS

The present invention provides those skilled in the art with a method for the diagnosis and monitoring of chronic gram-negative infections, particularly Pseudomonas aeruginosa infection associated with cystic fibrosis. The composition used in the present method comprises core lipopolysaccharide derived from Pseudomonas aeruginosa strain PAC 605, especially antigenic determinants found on such core lipopolysaccharide, and is employed in diagnostic methodology such as enzyme linked immunosorbant assay (ELISA) and Western blot assay.

The composition is employed because of its ability to bind immunoglobulin molecules (antibodies or active fragments thereof) which bind antigenic determinants on gram-negative bacteria. Donors of particular interest include human patients afflicted with cystic fibrosis. Immunoglobulin molecules of particular interest include human IgG molecules.

According to the method of the present invention, the antigenic determinant composition is bound to a solid support and employed in a variety of assay techniques well-known to the art, particularly ELISA, Western blot analysis, or the like. Solid supports of particular interest include a particle, e.g., bead, fiber, or the like, or container wall and include materials such as nitrocellulose, cellulose, agarose, polystyrene, dextran, glass, polymer coated magnetic metals, nylon, silica gel, polyacrylamide, polymethyl methacrylate, and the like, and of particular interest is nitrocellulose (NC).

The composition may be coupled directly to the support or via a spacer arm to aid in preventing any possible charge or steric interferences with antigen binding. Various spacer arms are available and well known in the art. The ratio of antibody to support may be about 100:1 usually about 1,000:1 or higher.

The following examples are offered by way of illustration.

EXPERIMENTAL

EXAMPLE I - MATERIALS AND METHODS

1. LPS: PAC 605 bacterial strain (parent strain PAC1R, Habs 0:3) is deposited with the American Type Culture Collection (A.T.C.C.), Rockville, MD, Accession No. 53273. Cells were grown in a aerated fermenter and LPS was extracted using the method described by Darveau and Hancock, J. Bacteriology (1983) 155:831-38, which is incorporated by reference. Coomasie blue dye protein assay (BIORAD) measured a protein contamination of 1%. Purified J5 E. coli, Fisher type 1 PA, and Salmonella minnesota Ra LPS were purchased from LIST Biological Laboratories.

2. Anti-PA core LPS monoclonal antibody: XMMPS-605 (A.T.C.C. Accession No. HB8909 is an IgG2b murine monoclonal antibody (MoAb). It was produced by fusing spleen cells with SP2/0 myeloma cells following immunization with killed PAC 605 cells. Hybrids were screened by using purified PAC 605 as antigen. XXMPS-605 is cross-reactive, binding to the core region of LPS from all 7 Fisher types of PA.

3. Patient sera: Normal human sera was donated by laboratory workers with no history of PA infection or of working with PA organisms. Patient sera was made available by the clinical laboratories of UCLA Center for the Health Sciences, Los Angeles, CA, and Pacific Medical Center, San Francisco, CA. Samples used were from excess serum obtained for routine chemistry and antibiotic levels.

4. IgG ELISA: An ELISA was developed and standardized to measure human IgG titer against purified PAC 605 LPS.

a) 25 $\mu$l of PAC 605 LPS (500$\mu$/ml in distilled water with 0.05% triethylamine (TEA), boiled for 2 hours before use) and 25 $\mu$l of O.02M MgCl$_2$ with 0.05% TEA were added to triplicate wells of polystyrene COSTAR 96-well EIA plates which where then covered and incubated overnight at 37°C.

b) Wells were washed three times with phosphate-buffered saline (PBS) with 0.02 M MgCl$_2$, pH 7.1. 100 $\mu$l of 1% gelatin in PBS/MgCl$_2$ was then added to LPS coated wells and triplicate control wells and incubated overnight at room temperature.

c) The next day 50 $\mu$l of human serum diluted serially in 0.1% gelatin in PBS/MgCl$_2$ was added to triplicate LPS coated and control wells and incubated for one hour. All plates had positive test controls (XMMPS-605 20 $\mu$g/ml) and negative test controls (pooled human serum from 4 volunteers, mixed just before use and diluted 1/640) added to triplicate LPS coated and control wells.

d) All wells were washed three times, then 50 $\mu$l of Protein A-peroxidase (BIORAD) diluted 1/10,000 in 0.1% gelatin in PBS/MgCl$_2$ was added to all wells and incubated for one hour.

e) Wells were washed 5 times, then 100 $\mu$l aliquots of ABTS® substrate was added to each well (per 5 ml: 4.9 ml 0.1 M disodium citrate buffer, pH 4.5; 0.1 ml of 2.2'-azino-d-[3-ethyl-benzthiazolinesulfonate {6}] (BOEHRINGER MANNHEIM), 20 mg/ml in citrate buffer; 5 $\mu$l 30% H$_2$O$_2$.

f) Optical density (O.D.) was read on an automated microreader (BIOTEK or FLOW LABORATORIES) when the O.D. of the positive plate control (XMMPS-605) was close to 1.0. Results of triplicate test and control O.D.'s were averaged. For a given test sample dilution, the mean control O.D. was subtracted from the mean test O.D. A positive serum titer was defined as that dilution yielding an O.D. greater than that of pooled human sera diluted 1/640 (negative control) recorded on the same plate.

5. Serum IgG levels: Patient and volunteer serum IgG levels were determined by radial immunodiffusion, using KALLESTAD IgG Endoplates. KALLESTAD low, medium, and high reference sera were used as controls.

6. SDS-polyacrylamide gel electrophoresis: PAC 605 and J5 E. coli purified LPS were fractionated on SDS-polyacrylamide slab gels with a discontinuous buffer system as described by Hames, B.D., Gel electrophoresis of Proteins: A Practical Approach, (1981) IRL Press, Oxford, England, P. 1-93. The separating gel contained 12.5% acrylamide/methylene bisacrylamide (30.0/0.8) in Tris/SDS buffer, pH 8.8. The stacking gel contained 4.5% acrylamide/methylene bisacrylamide in Tris/SDS buffer, pH 6.8. 350 $\mu$g of LPS was boiled for 10 minutes in final sample buffer and applied to a sample well. A lower molecular weight standard was applied to a control well. Gels were electrophoresed at 8 mAmps overnight, then at 18 mAmps until the tracking dye reached the bottom.

7. Electrophoretic transfer and immunoblot: Gels were immediately transferred to nitrocellulose for three hours at constant amperage (190 mAmp) using the Western blot method of Towbin et al., Proc. Nat. Acad. Sci. USA (1979) 76:4350-54. Molecular weight lanes were cut and stained with amide black in acetic acid and methanol. The remainder of the sheet was allowed to dry then stored at -20 degrees Centigrade until use. For immunoassay of these blots, 0.5 cm strips were cut and blocked for 30 minutes in 1% gelatin in PBS. Individual strips were then incubated on a rocker overnight with 4 ml of a 1/40 dilution of human serum in 0.1% gelatin in PBS. Positive control strips for each blot were incubated with either XMMPS-605, 20$\mu$/ml, or rabbit anti-J5 E. coli serum, 1/40 dilution. The following day strips were washed for one hour with 4 changes of PBS, then incubated in a 1/2000 dilution of Protein A-peroxidase, then washed for one hour with 6 changes of PBS. Strips were developed with 4 chloro-1-naphthol substrate (per 5 ml: 4 ml PBS, 1 ml of 0.3% w/v 4 chloro-1-naphthol [SIGMA] in reagent grade methanol, 5 $\mu$l 30% H$_2$O$_2$). When optimal color change had occurred, NC strips were washed in water.

8. Proteinase K digestion and periodate oxidation of LPS: To confirm that bands seen on immunoblots of LPS represent non-protein, carbohydrate determinants, 175 $\mu$g of PAC 605 LPS was pre-digested with Proteinase K (BRL) 1 mg/ml frozen stock added in a 1:10 v/v ratio to LPS in final sample buffer and incubated overnight at 55 degrees Centigrade. An additional aliquot of Proteinase K was added the next day and incubated for three more hours. This digested LPS was fractionated in one half of an SDS-polyacrylamide gel while an equal amount of undigested PAC 605 LPS was fractionated on the other half. SDS-PAGE and Western blot were performed as previously described. Strips containing digested and non-digested LPS were immunoblotted with positive patient sera.

Additional strips of blotted proteinase-treated PAC 605 LPS were oxidized with periodic acid to destroy carbohydrate epitopes, then immunoassayed using the same positive patient sera. Western blot strips of PAC 605 LPS and control immunodots of Salmonella minnesota Ra LPS were blocked in 1% gelatin in PBS, then bathed overnight at 4 degrees Centigrade in 50 mM periodic acid, 50 mM acetic acid, pH5. After brief washing, strips and control immunodots were immunoassayed as previously described. An IgG2a MoAb specifically reactive with Salmonella Ra LPS was strongly reactive against the control Ra immunodot but completely unreactive against the periodate-oxidized Ra LPS dot.

9. Silver stain: To confirm location of LPS bands, SDS-PAGE was done with individual lanes containing PAC 605 LPS, J5 E. coli LPS and several other PA LPS controls. This gel was then silver stained using the method described by Hitchcock and Brown, J. Bacteriology (1983) 154:269-77, which is incorporated by reference.

EXAMPLE II - RESULTS

1. IgG ELISA: Results of the ELISA used to detect human IgG antibodies to PAC 605 LPS are summarized in Tables 1, 2, and 3.

Convalescent sera were available for 10 patients with documented PA bacteremia. Five of these patients had documented localized infection prior to the onset of bacteremia (pneumonia [2], brain abscess [1], cystitis [1], sinusitis [1]). The geometric mean titer of thesse 10 patients (obtained a minimum of seven days after blood cultures were drawn) was 1/171 (range 1/40 - 1/1280). This did not differ significantly from the geometric mean titer of sera from 5 normal volunteers (mean 1/211, range 1/160 - 1/320, p> 0.5, Student's test) or of convalescent sera of 11 patients with documented non-PA enteric gram-negative bacteremia (mean 1/97, range 1/40 - 1/320, p> 0.5). Blood culture isolates from this latter group included E. coli (3), Klebsiella pneumonia (3), Enterobacter cloacae (3), Proteus mirabilis (2), and Aeromonas hydrophila (1).

## TABLE 1

### Geometric Mean IgG anti-PAC LPS Titers

| | Number of patients | Mean titer | Range | P value* |
|---|---|---|---|---|
| PA bacteremia, convalescent | 10 | 1/171 | 1/40-1/1280 | p> 0.5 |
| Non-PA gram-negative bacteremia, convalescent | 11 | 1/97 | 1/40-1/320 | p> 0.5 |
| Cystic fibrosis | 18 | 1/1808 | 1/320-1/10,000 | p< 0.001 |
| Normal volunteers | 5 | 1/211 | 1/160-1/320 | p> 0.5 |

*Student´s t test

6

## TABLE 2
### Paired Acute and Convalescent
### Mean IgG Anti-PAC 605 LPS Titers

| | Number of Patients | Acute | Convalescent | P value* |
|---|---|---|---|---|
| PA bacteremia | 6 | 1/143 | 1/160 | p> 0.5 |
| Non-PA gram-negative bacteremia | 11 | 1/132 | 1/97 | p> 0.5 |

*Student's t test

Paired acute and convalescent sera were available from 6 patients with PA bacteremia and all 11 patients with non-PA gram-negative bacteremia. Geometric mean acute and convalescent IgG titers did not significantly differ in either group. PA bacteremic patients had a mean acute titer of 1/143 (range 1/20 - 1/1280) and convalescent titer of 1/160 range 1/40 - 1/1280, p> 0.5). As mentioned above, 5 of the PA bacteremic patients had documented recent localized PA infection. Acute and convalescent titers were available for 4 of these 5 patients (Table 3). Three of these patients showed no increase in IgG anti-PAC 605 LPS titer, while one demonstrated a four-fold rise between acute and convalescent sera.

## TABLE 3

### Individual Acute and Convalescent IgG Anti-PAC 605 LPS Titers of PA Bacteremic Patients with Documented Prior, Localized PA Infection

| Source of prior localized infection | Acute titer | Convalescent* titer |
|---|---|---|
| Pneumonia | 1/160 | 1/80 |
| Pneumonia | 1/320 | 1/1280 |
| Sinusitis | 1/40 | 1/40 |
| Brain abscess | 1/160 | 1/160 |

*obtained at least 7 days after initial blood culture drawn

Random serum was also available from 18 patients with cystic fibrosis (CF) and chronic PA pulmonary infection. The geometric mean titer of these patients was significantly higher (mean 1/1808, range 1/320 - 1/10,000) than the mean titer of normal volunteers (p< 0.001) and the mean convalescent titer of patients with PA (p< 0.001) bacteremia.

IgG Levels: Hypogammaglobulinemia was excluded as the reason for the surprisingly low IgG anti-PAC 605 titer observed in convalescent sera of PA bacteremic patients compared to uninfected normal volunteers. Serum IgG levels were measured and compared. The mean IgG level of the 10 PA bacteremic patients (1,118 mg/dl, range 640-1680 mg/dl) did not differ significantly from the mean of 5 normal volunteers (1,236 mg/dl, range 1000-1380 mg/dl, p> 0.5). The CF patients' markedly higher IgG titer to PAC 605 LPS could have been a nonspecific artifact related to higher IgG levels. To test this hypothesis, 9 of the 18 CF patients' sera were randomly chosen and IgG levels were measured. The mean IgG level of these 9 patients was 1734 mg/dl, compared to 1118 mg/dl in the 10 PA bacteremic patients - not a significant difference (p> 0.1).

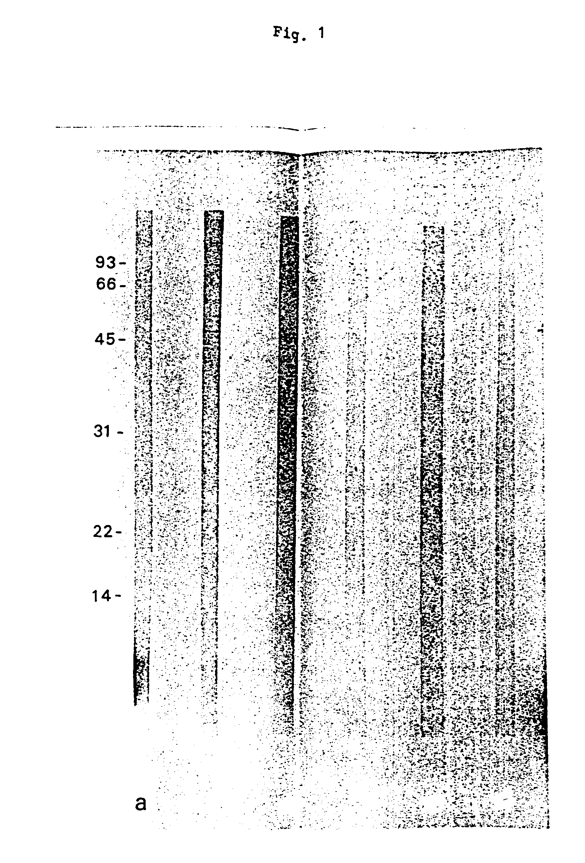

3. Western blots of PAC 605 LPS: In order to reveal the exact nature of the PAC 605 LPS determinants bound by human IgG, whole SDS-polyacrylamide gels were loaded with LPS, then fractionated by electrophoresis, then transferred by the Western blot method to nitrocellulose (NC) sheets. These sheets were cut into strips, incubated first with 1/40 patient sera, then with Protein A-peroxidase and finally developed with substrate. All data was confirmed by incubating the same test sera with a second strip blotted from a different prep gel of PAC 605 LPS. Results are summarized in Table 4. Several distinct band patterns were seen, and examples of each pattern are shown in Figure 1. As a positive control for each prep gel that was blotted, the murine monoclonal IgG2a designated XMMPS-605 (which is sensitive and specific for the core determinants of PAC 605 LPS) was reacted with one NC strip. This reproducibly showed an intense broad band between 10 kD and the dye front (see Fig. 1, lane A). No bands were visible when pooled normal human sera or convalescent sera of patients with PA bacteremia were incubated with strips of fractionated, blotted PAC 605 LPS. However 13 of 18 CF patients showed one of the following three patterns. Five patients demonstrated a single broad core band between 10 kD and the dye front (same pattern as that shown by the anti-PA core LPS monoclonal antibody XMMPS-605). Three patients showed a faint typical smooth LPS ladder pattern between 30 and 50 kD. Five patients showed both core and 30 - 50 kD ladder bands. One additional patient (RR) had a broad core band in combination with two narrow intense bands at 14 kD and 24 kD. When this immunoblot was repeated using PAC 605 LPS pre-treated with Proteinase K before electrophoresis, the 14 kD band disappeared suggesting it represented a protein determinant. Only 4 of the 18 had a low molecular weight core band and 8 of 18 had faint higher molecular weight ladder bands.

## TABLE 4
### Western Blot Band Patterns of
### PAC 605 LPS and J5 E. coli LPS

| PAC 605 LPS | Cystic fibrosis patient sera | PA bacteremic convalescent sera | P value * |
|---|---|---|---|
| Broad band | | | |
| < 10 kD | 11/18 | 0/10 | p<0.01 |
| Ladder bands, | | | |
| 30-50 kD | 8/18 | 0/10 | p<0.05 |
| | | | |
| J5 E. coli LPS | | | |
| Broad band | | | |
| < 10 kD | 11/17 | 3/9 | p> 0.1 |

*Chi square with Yate´s modification

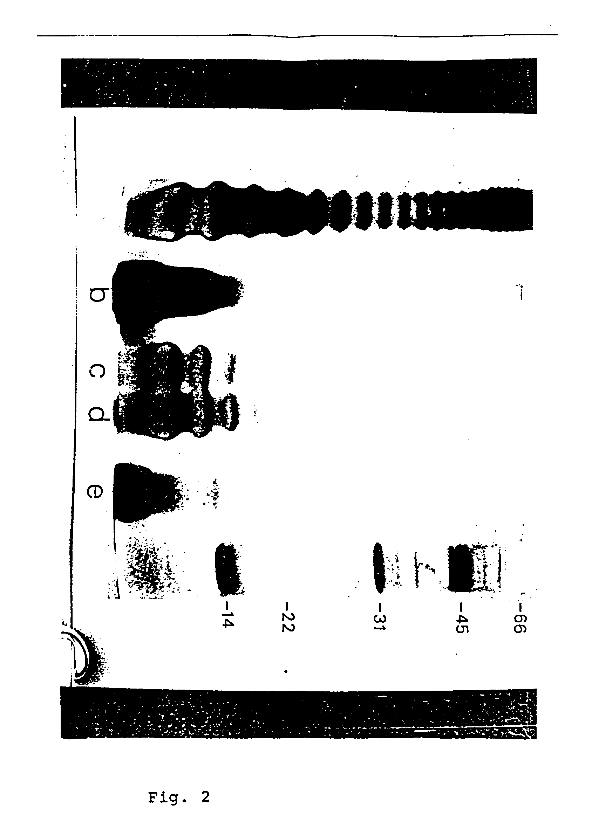

4. Silver stain of PAC 605 LPS: The appearance of 30 - 50 kD ladder bands was unexpected since PAC 605 LPS does not have smooth LPS. Several studies were done to elucidate the nature of these bands. PAC 605 LPS and several control purified LPS samples were fractionated by SDS-PAGE, then silver-stained to exclude the possibility of contamination. Twenty-five $\mu$g of PAC 605 LPS run in a single lane (Figure 2, lane E) showed an intense core band from 10 kD to the dye front - the exact location of the broad core band seen on Western blots reacted with both the anti-core MoAb XXMPS-605 and with 11 of the 18 CF patient sera. There were narrow sharp bands at 14 kD and 24 kD corresponding to the bands that occurred with CF patient RR's sera. The band at 14 kD may represent a protein-LPS complex since this band disappeared from RR's immunoblot when PAC 605 was pre-treated with Proteinase K. No bands were seen between 30 and 50 kD - the location of faint ladder bands on Western blot strips. As a control, Proteinase K digested boiled whole cells of a rough, non-typable sputum PA isolate from one of the CF patients were fractionated in the same gel (Fig. 2, lanes C and D). This apparently rough isolate has small amounts of high molecular weight smooth LPS faintly detectable when 7.5 x 108 cells were run in lane C, and clearly visible when 1.5 x 109 cells were run in lane D. Another gel run by SDS-PAGE was loaded with 25 - 125 µg of PAC 605 LPS per well. Silver stain of the overloaded wells showed a diffuse smear throughout the lane. Individual ladder bands, however, were not visible.

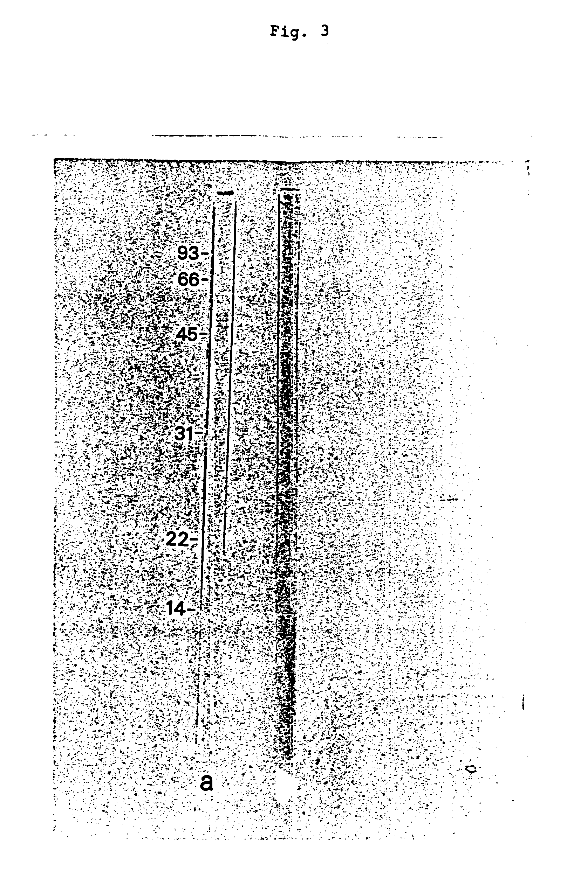

5. Proteinase K digestion and periodate oxidation of PAC 605 LPS: Immunoblots were repeated to determine the exact nature of these high molecular weight ladder bands. First PAC 605 LPS was pre-digested with Proteinase K to destroy any protein determinants before fractionating by SDS-PAGE and immunoblotting. NC strips of blotted, Proteinase K-digested LPS and untreated LPS were reacted with two CF patient's sera that had previously shown ladder bands (Figure 3, lane A). Ladder bands remained visible after Proteinase K treatment, suggesting the bands are not due to protein epitopes. Several of these LPS strips were then oxidized overnight with periodic acid, pH 5, to destroy carbohydrate determinants, as described by Caldwell and Hitchcock, Infect. Immun. (1984) 44:306-14, which is incorporated by reference, and then reacted with the same CF patient's serum. Ladder bands disappeared after periodate oxidation, suggesting that the responsible determinants are located on repeating polysaccharide units of smooth LPS.

6. Western blots of J5 E. coli LPS: If the core determinants of PAC 605 LPS are very similar to those of the cross-reactive core of enteric gram-negative bacilli (i.e. purified J5 E. coli LPS), then the difference in IgG anti-PA core LPS response between CF and convalescent PA bacteremic patients may just be a reflection of a difference in cross-reactive IgG anti-gram-negative core titer. To test this hypothesis, J5 E. coli LPS was fractionated by SDS-PAGE and Western blotted onto NC sheets. Individual NC strips were immunoassayed with sera from 17 CF patients and 9 convalescent PA bacteremic patients. All results were confirmed by incubating the same test serum with a second strip blotted from a different prep gel of J5 LPS. Eleven of 17 CF sera versus 3 of 9 convalescent PA bacteremic sera showed core bands below 14 kD (p> 0.1, Chi square with the Yates modification). These core bands were in the same location that immunoblotted rabbit anti-J5 LPS appeared on silver stain (Fig. 2, lane B). Hence it was highly unlikely that the difference between CF patients and convalescent PA bacteremic patients in IgG anti-PA core LPS response was simply due to cross-reactive antibodies to the common endotoxic core of enteric gram-negative bacilli.

## Claims

1. A method for detecting a chronic Pseudomonas aeruginosa infection in a donor by screening for an immunoglobulin molecule specific for an antigenic determinant on lipopolysaccharide derived from a Pseudomonas aeruginosa strain PAC 605 cell having A.T.C.C. Accession No. 53273, said method comprising the steps of:
   (a) contacting serum from said donor with said lipopolysaccharide; and
   (b) detecting a chronic Pseudomonas aeruginosa infection by determining whether the level of said immunoglobulin in said serum is significantly higher than the level of said immunoglobulin in serum from a donor known to be non-infected with Pseudomonas aeruginosa or in convalescent serum from a donor recovering from Pseudomonas aeruginosa infection.

2. The method according to claim 1, wherein said donor is a human.

3. The method according to claim 1 or 2, wherein said immunoglobulin is a class IgG.

4. The method according to anyone of claims 1 to 3, wherein said lipopolysaccharide comprises a core antigenic determinant.

5. The method according to anyone of claims 1 to 4, wherein said lipopolysaccharide is bound to a support prior to contacting with said serum.

6. The method according to anyone of claims 1 to 5, wherein the donor of the serum is a patient having cystic fibrosis.

## Patentansprüche

1. Verfahren zum Feststellen einer chronischen Pseudomonas aeruginosa-Infektion in einem Spender durch Screenen auf ein Immunoglobulinmolekül, das für eine Antigen-Determinante auf einem Lipopoly-

EP 0 265 672 B1

saccharid spezifisch ist, welches von einer Pseudomonas aeruginosa-Stamm-PAC 605-Zelle mit der A.T.C.C.-Zugriffsnummer 53 273 abgeleitet ist, das folgende Schritte umfaßt:

a) Inkontaktbringen von Serum von dem Spender mit dem Lipopolysaccharid; und

b) Feststellen der chronischen Pseudomonas aeruginosa-Infektion durch Bestimmung, ob der Spiegel des genannten Immunoglobulins in dem Serum signifikant höher ist als der Spiegel des genannten Immunoglobulins in einem Serum von einem Spender, von dem bekannt ist, daß er nicht mit Pseudomonas aeruginosa infiziert ist, oder von einem Rekonvaleszenten-Serum von einem Spender, der sich von einer Pseudomonas aeruginosa-Infektion erholt.

2. Verfahren nach Anspruch 1, in dem der Spender ein Mensch ist.

3. Verfahren nach Anspruch 1 oder 2, in dem das Immunoglobulin von der Klasse IgG ist.

4. Verfahren nach einem jeden der Ansprüche 1 bis 3, in dem das Lipopolysaccharid eine Kern-Antigendeterminante umfaßt.

5. Verfahren nach einem jeden der Ansprüche 1 bis 4, in dem das Lipopolysaccharid an einen Träger vor dem Inkontaktbringen mit dem Serum gebunden wird.

6. Verfahren nach einem jeden der Ansprüche 1 bis 5, in dem der Serumspender ein Patient mit cystischer Fibrose ist.

**Revendications**

1. Procédé pour la détection, chez un donneur, d'une infection chronique par Pseudomonas aeruginosa, au moyen d'un criblage révélant la présence d'une immunoglobuline spécifique d'un déterminant antigénique porté par un lipopolysaccharide dérivé de la souche de Pseudomonas aeruginosa PAC 605 et ayant le numéro d'accès 53273 auprès de l'A.T.C.C., ledit procédé comprenant les étapes suivantes :

a) la mise en contact du sérum provenant dudit donneur avec ledit lipopolysaccharide ; et

b) la détection d'une infection chronique par Pseudomonas aeruginosa en déterminant si le taux de ladite immunoglobuline présent dans ledit sérum est sensiblement plus élevé que le taux de ladite immunoglobuline dans le sérum d'un donneur non infecté par Pseudomonas aeruginosa, ou dans un sérum convalescent provenant d'un donneur se rétablissant d'une infection par Pseudomonas aeruginosa.

2. Procédé selon la revendication 1 dans lequel ledit donneur est un être humain.

3. Procédé selon la revendication 1 ou 2 dans lequel ladite immunoglobuline appartient à la classe IgG.

4. Procédé selon l'une quelconque des revendications 1 à 3 dans lequel ledit lipopolysaccharide comprend un déterminant antigénique du noyau.

5. Procédé selon l'une quelconque des revendications 1 à 4 dans lequel on fixe ledit lipopolysaccharide à un support avant de le mettre en contact avec ledit serum.

6. Procédé selon l'une quelconque des revendications 1 à 5, dans lequel le donneur du sérum est un patient atteint de mucoviscidose.

11

Fig. 1

Fig. 2

EP 0 265 672 B1

Fig. 3

14

Fig. 4